# EUROPEAN PATENT APPLICATION

(11) **EP 4 385 531 A1**
(43) Date of publication of application: **19.06.2024**
(21) Application number: 22213996.6
(22) Date of filing: 15.12.2022
(51) Int. Cl.: A61L 27/18, A61L 27/20, A61L 27/22, A61L 27/24, A61L 27/38

(54) **PROCESS FOR MANUFACTURING A MARTIX FOR BIOMEDICAL APPLICATIONS**

(71) Applicant: Healiva SA, 6900 Lugano (CH)
(72) Inventor: DUTTA PASSECKER, Priyanka, 1090 Vienna (AT); ROLLAND, Eric, 1400 Yverdon-les-Bains (CH); EMMENDöRFFER, Andreas, 4332 Stein (CH); SIDDHARTH, Jay, 6816 Bissone (CH); RIMANN, Markus, 8006 Zürich (CH); BONO, Epifania, 8805 Richterswil (CH)
(74) Representative: M. Zardi & Co S.A.

(57) **Abstract**

A new method to obtain a matrix loaded with blood cells is herein disclosed. The method comprises the steps of: (a) providing: a liquid phase containing blood cells; a solid matrix connecting said liquid phase with a positive electrode; a solid matrix connecting liquid phase with a negative electrode; and (b) applying an electric field between positive and negative electrodes, resulting in a movement of blood cells towards one of the electrodes and in cell-loading of the corresponding matrix. The method results in efficient and homogeneous incorporation of the blood cells into the solid matrix by electrical field application. The invention includes the matrix obtained by this method and its medical use, in particular for wound healing and tissue regeneration purposes.

## Description

### FIELD OF THE INVENTION

The invention relates to i) the field of biocompatible supports for human or animal application for therapeutic purposes and to ii) methods for their preparation. In particular, it relates to the preparation of selected cell-containing matrices and to the use thereof for medical purposes, including tissue repair and/or tissue regeneration.

### BACKGROUND ART

Tissue repair and wound healing are naturally occurring biological processes triggered by the presence of an injury on human or animal tissues. These processes take place via several phases such as hemostasis, inflammation, proliferation, and remodeling: they involve the activation of inflammatory factors and enzymes, the elimination of injured cells, the formation of new cells, the formation of a cicatrizing tissue, angiogenesis, etc. An important branch of medical innovation is devoted to promoting the speed and efficiency of these processes for patient's benefits, with an additional view to protect the injured/regenerating tissues from possible infection.

Medical products for application to the injured tissue, including regular dressings, advanced dressings, physical treatments, advances wound-healing treatments or growth factors, are known. The advanced dressings are generally made of a biocompatible porous material or a biopolymer aimed to facilitate the healing process. In some cases the matrices could contain active factors (drug, cells having a beneficial effect on the tissue regeneration process, etc). Among cells, platelets and/or leucocytes are frequently used for wound healing purposes.

The drugs are generally water-soluble molecules which are easily loaded in the matrix and slowly released from the matrix applied to the injured tissue. Cells, however, have a much higher size/mass and therefore it is more difficult to disperse them, homogeneously and in high amounts, throughout the whole matrix; moreover, it is mandatory and crucial that cells maintain their viability and functionality (biological activity), which poses limits to the applicable loading techniques, might compromise cell viability.

Centrifugation is used in principle to incorporate a non-soluble material into a solid matrix: accordingly, a liquid medium containing the solid matrix and the non-soluble material in suspension is subjected to centrifugation, where the applied G-force drives the particles of non-soluble material into the matrix. However, by such a technique, the cells may impact rapidly and massively onto the surface of solid biomaterial where they tend to accumulate and stratify. On the opposite side, a simple soaking up of the biomaterial into a liquid containing the cells to be loaded, in absence of centrifugation, would also be inefficient, since the process is lengthy and may result in superficial saturation areas which oppose to a further penetration of the cells into the deeper portions of the biomaterial.

The patent application EP 2 334 307 describes a multilayered blood product containing fibrin, thrombocytes and leukocytes separated in three layers; the product is obtained from a patient's blood, where separation and stratification of the blood components and solidification is obtained by centrifugation, coagulation, and compaction. The related application EP 2 618 865 describes a plastic polymeric container suited for the preparation of multi-layered blood product. Products obtained with this technology are distributed with the commercial name of 3C Patch^{®}. These approaches concern the separation of the blood components and do not deal with their incorporation into a matrix.

Lin et al. (Journal of Immunology, 2008; 181(4): 2465-2471)) show that an electric field can induce lymphocytes directional migration in vitro as well as in vivo.

In view of the reviewed art, there is high need for matrices characterized by a high and homogeneous incorporation of cell components and we need new technical methods capable to obtain this. The electric field application on a system containing blood cells and a matrix could represent the solution i) for the homogenous loading of selected blood cells in a biomaterial and ii) for the maintenance of high cell viability and functionality (biological activity).

### SUMMARY OF THE INVENTION

A new method to obtain a matrix loaded with blood cells is herein disclosed. The method comprises the steps of: (a) providing: a liquid phase containing the blood cells; a solid matrix connecting said liquid phase with a positive electrode; a solid matrix connecting said liquid phase with a negative electrode; and (b) applying an electric field between said positive and negative electrodes, resulting in a movement of blood cells towards one of the electrodes and in cell-loading of the corresponding matrix. The method results in efficient and homogeneous incorporation of blood cells into the solid matrix by electrical field application. The invention includes the matrix obtained by this method and its medical use, in particular for the wound healing and tissue regeneration purposes.

In a more detailed embodiment, the method is performed as follows:
(a) providing:
   - a first compartment containing said liquid phase comprising the blood cells,
   - a second compartment containing said positive electrode immersed in an electrolyte fluid,
   - a third compartment containing said negative electrode immersed in an electrolyte fluid,
   - a solid matrix partly immersed in the liquid phase and partly immersed in the electrolyte fluid of the first compartment, thereby forming a bridge between the liquid phase and the electrolyte fluid of the first compartment;
   - a solid matrix partly immersed in the liquid phase and partly immersed in the electrolyte fluid of the second compartment, thereby forming a bridge between the liquid phase and the electrolyte fluid of the second compartment;
(b) applying an electric field between said positive and negative electrodes, resulting in a movement of blood cells towards one of the electrodes and in cell-loading of the corresponding matrix.

### BRIEF DESCRIPTION OF THE DRAWINGS

Figure 1: shows a system on which the process of the invention can be performed: the system employs a 2-electrode system for loading test cells to be subjected to an electric field.
Figure 2: Trajectory plot of erythrocytes in a leucocyte enriched fraction of human blood sample with application of electric field (EF, left plot) and without EF application (right plot). Cells were tracked manually with ImageJ and results were plotted with the software "Chemotaxis and Migration Tool" supplied by iBidi.

### DETAILED DESCRIPTION OF THE INVENTION

The liquid phase used in the present system can be made of any liquid compatible with the viability of blood cells: typically, it is an aqueous electrolyte solution, preferably a physiological saline solution, such as e.g., a phosphate-buffered saline (PBS). Alternatively, the liquid phase is a culture medium for blood cells or a human/animal biological liquid which physiologically carries blood cells, in particular human/animal serum or an aqueous solution thereof, or heparinized whole blood. Aqueous solutions containing 5-30 wt % of human serum have been found particularly effective in increasing the blood cell speed of migration. Optionally, the heparinized blood is diluted with a physiological liquid, e.g. a saline solution, in order to reduce its density (viscosity) and to improve cell migration movement. In another embodiment, the liquid phase is a heparinized blood fraction enriched with the blood cells of interest; to this purpose, for example, heparinized blood can be mixed with a suitable hydrocolloid like hydroxyethyl starch: after a suitable time e.g. 30 minutes at room temperature, two separate phases are formed, respectively enriched with leukocytes and erythrocytes.

The term "solid matrix" used herein comprises any substance which, at room temperature, is insoluble in water but penetrable by water; the term "solid" includes also substances in the gelled form or in any other physical form which maintains its integrity in contact with water; the solid matrix has preferably a porous or microporous structure in which cells can be loaded and incorporated. The term "solid" defining the matrix includes substances which are solid when applied to the human body but may progressively be digested and eventually dissolved on contact with the body fluids.

The material making up the solid matrix is generally a biopolymer compatible with human/animal tissues; it can be of natural, semi-synthetic or synthetic origin; non-limiting examples thereof are: agarose, hyaluronic acid, starch, alginates, collagen, fibrin, actin, gelatin, cellulose, methylcellulose, carboxymethylcellulose, chitosan, polyvinylpyrrolidone, polylactide, polyglycolide, polylactide/polyglicolide copolymers, polyethylene glycol, polyglycerol, polycaprolactone, etc., and combinations thereof. Commercially available matrices known as Hyalomatrix, Tisseel, Tissucol, etc. can also be used.

In the present method, the liquid phase containing the blood cells is placed in contact with the positive and negative electrode via the matrices. Said contact is preferably obtained by immersing one part of a matrix into said liquid phase and another part of the same matrix in an electrolyte fluid into which the negative (or positive) electrode is immersed. A closed system is thus obtained where the liquid phase containing the cells is connected, via matrices, to the electrolyte fluids containing the electrodes. The closed system allows the application of an electric field and the flow of a current.

The electrodes used in the process can be two or more in number, including at least one cathode and one anode. When more than two electrodes are present, they are preferably chosen to obtain the highest possible number cathode/anode couples, in order to maximize the strength and extension of the generated electric filed(s). In these cases, various arrangements of electrodes can be envisaged: for instance, two opposing rows of electrodes, respectively cathodes and anodes, can be construed such that, on application of the electric field, an overall cell migration takes place between the two rows. Alternatively, one or more rows can be set up, each made of alternating cathodes and anodes, wherein the application of the electric filed generates sub-migrations of cells between each couple of opposite adjacent electrodes. In other arrangements, in place of rows, the electrodes can be disposed in semi-circular or circular forms, or in combined row/circular arrangements, without limitation. The opposite electrodes of each couple are connected with each other via a power supply.

The electrodes are made of conventional materials used in the preparation of anodes (e.g. lithium, silver, tin, graphite, etc.) and cathodes (e.g. nickel, manganese, silver, cobalt, aluminum oxides, etc.). A typical electrode is Ag/AgCl. In the present system, the electrodes are put in contact with the liquid phase containing the blood cells; the contact is obtained via a the solid matrix, though which the cells can migrate; in this setup, in order to reach the target electrode, the migrating cells pass through the matrix, which is accordingly loaded with the cells.

In the present process, the system prepared as described above is subjected to an electric field obtained by applying a potential difference between the cathode(s) and anode(s): the electric field induces a migration (galvanotaxis) of the cells present in the liquid phase, preferably towards the anode. The potential difference applied to the electrodes and thus the electric field established between them can be varied in intensity and time duration depending on the nature and amount of the cell to be infiltrated in the matrix. A convenient working range for the applicable difference of potential can be, without limitation, comprised in the range of 100 - 1000 mV/mm, preferably 150 - 400 mV/mm, applied for a time comprised between 10 minutes and 7 hours, preferably between 1 and 5 hours. The electric field can be further characterized by an intensity comprised in the range of 0.5 - 2.0 Ampere, preferably 0.7 - 1.0 Ampere. The selected features of the electric field (potential, intensity) are not necessarily maintained constant during the process, but can be varied on need, modifying accordingly the loading process.

In the present system, the solid matrix is immersed in the liquid phase in a position suitable to be exposed to the generated electrical field. Specifically, the solid matrix is interposed between at least one cathode and at least one anode of the system, thus efficiently intercepting the cell migration flux generated by the electric field. Upon intercepting the solid matrix, the migrating cells penetrate it in depth, preferably via the pores of the matrix. Preferably the pores have average size higher than that of the migrating cells; in this case the migration through the matrix stops when the application of a potential difference between the electrodes is interrupted; however it is also possible to envisage a matrix having a gradient of pore sizes, which decreases moving from the surface to the inner sections of the matrix; in this case, the electric field-driven migration of the cells within the matrix continues until the size of its pores narrows below the average cell size, at which moment the cell is framed into the matrix.

In the present process, the cell migration is promoted by the electric field, such that the cells have sufficient kinetic energy to penetrate even the deeper layers of the solid matrix; the migration process is also prolonged in time, such that the intercepted cells have sufficient time to penetrate the inner layers of the solid matrix, without crowding on its outer surface and forming clots of cells opposing to a deeper loading of the structure. A consistent, homogeneous loading of the solid matrix is thus obtained. Furthermore, the application of the electric field and the associated loading into the solid matrix do not compromise or reduce the viability and functionality (biological activity) of the blood cells, such that the biological activity of the loaded matrix remains at optimal levels.

All types of blood cells can be used in the present process to obtain correspondingly loaded matrices. Preferably, they are human blood cells. The blood cells can be leukocytes (white blood cells, including macrophages), erythrocytes (red blood cells) or platelets; leucocytes and platelets are preferred for matrices aimed to wound healing/tissue regeneration, since they play a key role in these processes. The leukocytes can be: monocytes, lymphocytes, neutrophils, eosinophils, basophils and/or macrophages. The chosen blood cells may be used in the present process in mixed form with other blood cells, e.g. as they are present in blood, or they can be used as purified fraction which contains them in enriched form or in selective form, i.e. in absence of different blood cell types.

A further object of the present invention is a matrix loaded with blood cells, as obtained by the process described above. The matrix is characterized by a high level of viable cell loading, joined to a uniform distribution of cells throughout its structure.

In addition to blood cells, the matrix can be optionally loaded with other bioactive agents which can cooperate and possibly synergize with the blood cells in the desired application, particularly in wound healing and/or tissue regeneration. Examples of these additional active agents are: Allantoin, Bacitracin, Behenyl alcohol (docosanol, Abreva), Benzocaine, Cadexomer iodine, Calamine, Chlorhexidine, Dimethicone, Hydrocortisone, Hydrogen peroxide, Iodine, Iodoform, Lidocaine, Manganese chloride, Polymyxin B sulfate, Potassium iodide, Povidone iodine, Povidone USP (Plasdone K 29-32), Salicylic Acid, Silver sulfadiazine, Sodium fluoride, Thrombin, Tromethamine USP, White petroleum, Acesulfame K, Activated charcoal, Aluminum hydroxide, Aluminum oxide, Aluminum sulfate, Ammonium phosphate, Ascorbyl palmitate (Vitamin C ester), Beeswax, Benzalkonium chloride, Benzoic acid, Benzyl alcohol, Bismuth subgallate, Butylated Hydroxytoluene (BHT), Butylene glycol, Calcium, Calcium carbonate, Calcium chloride, Calcium oxide, Calcium sulfate, Candelilla wax, Cetearyl alcohol (Cetostearyl alcohol), Ceteth-20, Cetyl alcohol, Cetyl palmitate, Cholesterol, Citric acid, Copper, Cyclodextrin, Dehydroacetic acid, Diazolidinyl urea, Diisopropyl adipate, DMDM hydantoin, Ferric chloride Hexahydrate, Ferric oxide, Glycerin (glycerol), Glyceryl monostearate, Glyceryl stearate, Hydrochloric acid, Hydrogenated castor oil, Hydrogenated lecithin, Hydroquinone, Hydrous lanolin, Iron sulfate, Isopropyl alcohol, Isopropyl myristate, Kaolin, Lactic acid, Lecithin, Light mineral oil, Magnesium aluminum silicate, Magnesium oxide, Magnesium stearate, Magnesium sulfate, Malic acid, Maltodextrin, Mannitol, Menthol, Methyl salicylate, Methylene blue, Mineral oil, Palmitic acid, Parabens (various forms), Paraffin, Pentalyn-H (Pentaerythritol ester of rosin), Petrolatum, Phenoxyethanol, Phosphoric acid, Potassium sorbate, Propyl gallate, Propylene glycol, Rubidium chloride, Saccharin, Sodium benzoate, Sodium citrate, Sodium lactate, Sodium metabisulfite, Sodium sulfate, Sorbic acid, Sorbitan sesquioleate (Arlacel C), Sorbitol, Squalane, Steareth-10, Stearic acid, Sucrose, Sucrose laurate, Tartaric acid, Titanium dioxide, Triethanolamine (TEA), Trolamine, Vitamin C (ascorbic acid), Vitamin E (tocopherol), Xanthan gum, Xylitol, Zirconium oxide, Acetic acid, Alcohol, Copper chloride (cupric chloride), Crystal violet, Ethanol, Gentian violet, Germaben II, Hypochlorous acid, Liquid Germall Plus (propylene glycol, diazolidinyl urea, iodopropynyl butylcarbamate), Ozone, Polyaminopropyl biguanide (PAPB), Polyhexamethylene biguanide (PHMB, polyhexanide), Polyvinyl pyrrolidoneiodine, Quaternium 15, Silver (various forms), Sulfur dioxide, Triiodide resin, Zinc (various forms), African palm oils, Almond meal, Aloe vera, Angelica sp., Aqueous wheat extract, Avocado oil, Bisabolol (chamomile oil), Borneol, Butyrospermum parkii, Camella sinensis, Carvacrol, Centella asiatica, Citris grandis extract, Cocoamphodiacetate, Cupuacu butter, Eucalyptus oil, Eugenol, Extracts of licorice (deglycyrrhizinated), Fruit extract, Glycyrrhetinic acid (licorice extract), Guar gum (Cyaiuopsis letragonolobus), Gum mastic, Hydroxypropyl guar, Karaya gum, Konjac flour, Lavender, Lemon, Meadowsweet extract, Myristyl myristate, Myrtillus extract, Oak extract, Oat glucan, Olive oil, Palm glycerides, Piroctone olamine, Polygonum cuspidatum, Sandalwood oil, Shea butter, Solanum lycopersicum (tomato) extract, Soy protein, Styrax, Tara Gum, Tea tree oil, Theobroma Grandiflorum seed butter, Thymol, Transcinnamaldehyde, Vaccinium (blueberry), Vegetable oil, Vitis vinifera (grape), Wintergreen fragrance, Wood pulp core, Acetamide MEA (monoethanolamine), Aluminum magnesium hydroxide stearate, Aluminum pigment, Arachidyl alcohol, Ascorbyl tetraisopalmitate (Vitamin C ester), Betaines (various forms), Bismuth tribromophenate, Capryloyl glycine, Ceramide, Ceteareth-10 phosphate, Cetyl dimethicone copolyol, Chlorine dioxide, Chlorophyllin copper complex sodium, Chromium chloride, Cobalt chloride, Colloidal silica, Conjugated linoleic acid, Cyclomethicone, DEA Cetyl phosphate, Decanoic acid (capric acid), Dialkyl carbamoyl chloride, Dicetyl phosphate, Dipolyhydroxystearat e, Dissolved oxygen, EDTA, Ethoxydiglycol, Ethylene glycol monostearate, Ethylhexyl glycerin, Ethylhexyl palmitate, Fumed silica, Glyceryl monolaurate, Hectorite clay, Hexyl laurate, Hydroxypropyl bispalmitamide MEA (ceramide), Iron (various forms), Isohexadecane, Isopropyl sorbate, Keratin, L-glutamic acidLyophilized formulate porcine plasma, Manganese oxide, Methyl triethoxysilane (MTES), Methylal, Molybdenum chloride, O-cymen-5-ol (Biosol), Palmitamide MEA, Panthenol FCC (form of vitamin B), Pentylene glycol, Phosphorus pentoxide, Polyricinoleate, Potassium ferrate, Potassium iron oxyacid salt, Pyroglutamic acid, RADA-16 peptide, Sarcosine, Sodium selenite, Sodium tetraborate (Borax), Sucralfate (sucrose octasulfate, aluminum hydrochloride), Telmesteine, Titanium oxide, Tonalin FFA 80, Triglycerol (polyglycerol-3). etc., and combinations thereof. The loading with the other bioactive agents can be obtained by known means, e.g. soaking the matrix into a solution of the corresponding additional agent, etc.; alternatively, the present method can also be used to load an additional agent, insofar as the corresponding molecule is susceptible to galvanotaxis.

An even further object of the invention is the use of the above described matrix in medicine, in particular for wound healing and/or tissue regeneration purposes. The matrix is thus usable in a method of wound healing and/or tissue regeneration, characterized in that an effective amount thereof is applied to the tissue of a human patient or animal in need thereof. The tissue can be any tissue in need of treatment, for instance skin, connective tissue, derma, mucosa, etc.; surface tissues of the patient (skin, externally accessible mucosae e.g. mouth mucosa) can be treated by coating the treatable area with the matrix of the invention; non-superficial tissues (e.g. derma, connective tissue, internal mucosae) can be treated by transdermal application of the matrix or by injecting the same at the proper depth below the skin.

The tissue to be treated can be in an injured state or in any other condition in which the promotion of tissue regeneration is deemed beneficial; the type of matrix (polymer and its properties) will be suitably chosen in function of its compatibility with the tissue to be treated. The matrix can be applied to the tissue as such; alternatively, it can be incorporated into a suitable solid carrier support, e.g. a dermal patch or semi-solid patch, which enables its handling by the user and its application onto the tissue in need. The carrier support may include adhesive portions aimed to come into contact with areas adjacent to the site in need of treatment, thus ensuring a firm and precise positioning of the matrix with respect to the site in need of treatment.

The present invention is now disclosed by means of the following non-limiting example.

### EXAMPLE

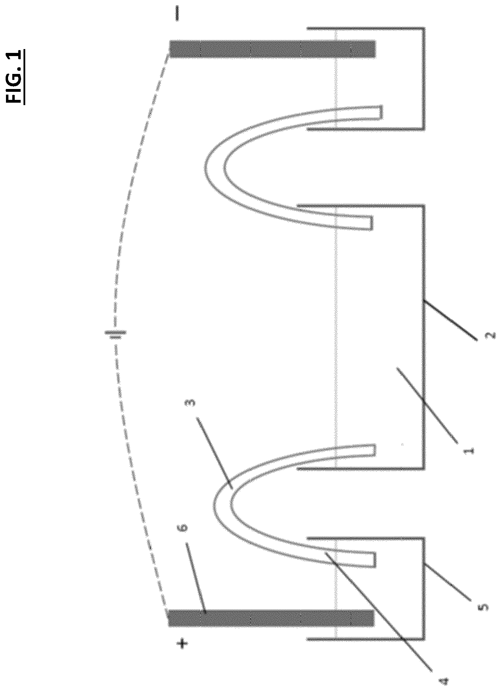

A matrix-loading system was prepared according to the assembly shown in Figure 1. A liquid medium (1) containing loadable test cells (mouse macrophages) was introduced into a suitable container (2). Two agarose bridges (3), (3') were immersed at one extremity into the liquid medium; the respective non-immersed extremities (4), (4') of the two bridges were introduced into corresponding containers (5), (5') containing a electrolyte (NaCl) solution; a positive electrode (6) (Ag/AgCI) was introduced into the solution of container (5) and a negative electrode was introduced into the solution of container (5'). An electric field of 300 mV/mm was applied for a period of 2 hours. Cell migration towards the anode was observed by microscope observation, resulting in loading of the interposed agarose rod with the cells. The loading was found to be substantial and, in a subsequent experiment, the viability of the loaded cells was assessed and found to be maintained.

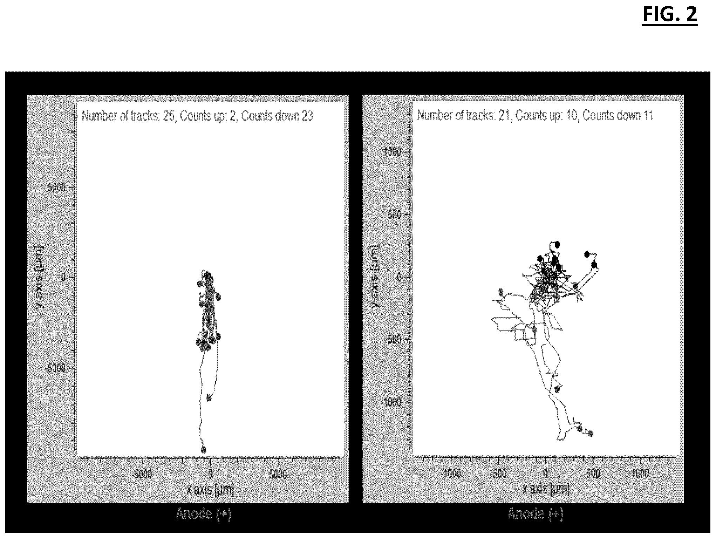

Further experiments were performed on a leucocyte enriched fraction of human blood sample to evaluate the blood cell mobility features for the purpose of migration into a polymer matrix. The mobility was studied with application of an electric field (EF) 230-320 mV/mm, 0.7-1 mA, cf. Fig. 2 left plot) and, as a reference, without EF application (cf. Fig. 2 right plot). Cells were tracked manually with ImageJ and results were plotted with the software "Chemotaxis and Migration Tool" supplied by iBidi. As evident in the left plot of Figure 2, a clear migration of cells towards the anode was detected on application of the EF, while for the untreated reference (right plot) the movement was slower and randomly directed. Another useful effect is the orientation of migration, whereby the application of the EF promotes a directionally selective movement of the whole cell population, i.e. the cells move orderly towards the target as one compact group, whereas the reference untreated cells move randomly in the available space. The selective directionality of migration obtained by the invention permits, when preparing the galvanotaxis apparatus, to place the solid matrix in the most suitable position to intercept the stream of migrating cells, thereby maximizing loading efficiency and yield.

## Claims

1. Method to obtain a matrix loaded with blood cells comprising the steps of:
(a) providing:
(i) a liquid phase containing blood cells;
(ii) a solid matrix connecting said liquid phase with a positive electrode;
(iii) a solid matrix connecting said liquid phase with a negative electrode;
(b) applying an electric field between said positive and negative electrodes, resulting in a movement of blood cells towards one of the electrodes and in cell-loading of the corresponding matrix.

2. Method according to claim 1, comprising the steps of:
(a) providing:
- a first compartment containing said liquid phase comprising the blood cells,
- a second compartment containing said positive electrode immersed in an electrolyte fluid,
- a third compartment containing said negative electrode immersed in an electrolyte fluid,
- a solid matrix partly immersed in the liquid phase and partly immersed in the electrolyte fluid of the first compartment, thereby forming a bridge between the liquid phase and the electrolyte fluid of the first compartment;
- a solid matrix partly immersed in the liquid phase and partly immersed in the electrolyte fluid of the second compartment, thereby forming a bridge between the liquid phase and the electrolyte fluid of the second compartment;
(b) applying an electric field between said positive and negative electrodes, resulting in a movement of blood cells towards one of the electrodes and in cell-loading of the corresponding matrix.

3. The method according to claims 1-2, wherein the liquid phase is selected from, a physiological saline solution, blood serum or aqueous solution thereof, heparinized blood, or a solution enriched with/selectively containing one type of blood cells selected from leukocytes, platelets, or erythrocytes.

4. The method of claim 3, wherein the heparinized blood is diluted in a saline solution.

5. The method of claim 4, wherein the heparinized blood is diluted in the solution at a w/w concentration ranging from 1:3 to 1:150.

6. The method according to claims 1-5, wherein matrix comprises one or more among agarose, hyaluronic acid, starch, alginates, collagen, fibrin, gelatin, cellulose, methylcellulose, carboxymethylcellulose, chitosan, polyvinylpyrrolidone, polylactide, polyglycolide, polylactide/polyglicolide copolymers, polyethylene glycol, polyglycerol and polycaprolactone.

7. The method according to claims 1-6, wherein the electric field is **characterized by** a potential of 100-1000 mV/mm.

8. The method according to claims 1-7, wherein the electric field is **characterized by** an intensity of 0.5-2.0 Ampere.

9. The method according to claims 1-8, wherein the electric field is applied for a time ranging from 10 minutes and 7 hours.

10. Matrix loaded with blood cells, obtained by the method of claims 1-9.

11. The matrix according to claim 10, further including one or more additional substances active on wound healing and/or tissue regeneration.

12. The matrix of claims 10-11 for use in wound healing and/or tissue regeneration methods.
